# EUROPEAN PATENT SPECIFICATION

(11) **EP 0 662 007 B1**
(45) Date of publication and mention of the grant of the patent: **30.07.1997**
(21) Application number: 93921005.0
(22) Date of filing: 27.09.1993
(51) Int. Cl.: A61M 5/145

(54) **FLUID DELIVERY APPARATUS**
FLÜSSIGKEITSABGABEVORRICHTUNG
APPAREIL DE DISTRIBUTION DE FLUIDE

(30) Priority: 25.09.1992 AU PL4922/92; 08.02.1993 AU PL7134/93; 15.07.1993 AU PL9952/93; 17.09.1993 AU PM1256/93
(43) Date of publication of application: 12.07.1995
(60) Divisional application: 96118673.1
(73) Proprietor: O'NEIL, Alexander George Brian, Subiaco 6008, Perth, W.A. (AU); O'NEIL, Christine, Subiaco 6008, Perth, W.A. (AU)
(72) Inventor: O'NEIL, Alexander, George, Brian, Perth, W.A. (AU)
(74) Representative: Pattullo, Norman
(86) International application number: GB9302009
(87) International publication number: WO9407551

(56) References cited:
- EP-A- 0 435 512
- WO-A-91/06338
- FR-A- 2 131 077
- GB-A- 1 105 820
- US-A- 4 744 786

## Description

This invention relates to fluid delivery apparatus.

Conventional pumps used in the medical device industry are primarily electronically controlled and electronically driven. While the industry has focused in this direction there are many disadvantages of electronics. These include the risk of microelectric shocks, variations in power supplies, lack of batteries and cost.

In addition, a number of spring driven syringes have been marketed together with fine port tubing to control the flow rate of fluid. All of these spring driven systems provide a fixed pressure profile and a fixed flow rate controlled by the flow control tubing. They are viscosity dependent and temperature dependent.

An apparatus having the pre-characterising features defined in claim 1 is disclosed in GB-1 105 820.

According to the present invention there is provided apparatus for delivery of fluid to a patient, the apparatus comprising a first reservoir for containing a pressurized first fluid, a second reservoir for containing the fluid to be delivered and having an outlet therefor, means between the first and second reservoirs for transferring a force produced by the pressure of the first fluid to the fluid of the second reservoir, characterised in that a tube having a narrow lumen is disposed between the outlet of the second reservoir and the patient, and where the rate of fluid delivery to the patient is determined by the diameter of the lumen of the tube.

Preferably, the first reservoir has an inlet for receiving said first fluid, the inlet being in communication with a pressurised source of said first fluid. The first fluid is preferably a gas, for example air.

Preferably also, the second reservoir comprises a cylinder and said means for transferring a force comprises a piston movable therealong.

Further preferably, the first reservoir comprises a cylinder and said means for transferring a force comprises a piston movable therealong. The cylinders of the first and second reservoirs may be for example in the form of syringes, and they may be connected together in tandem; this may be achieved by means of a connector member which engages each of the cylinders through a bayonet-type fitting.

It is of especial advantage in the present invention for the force produced by the pressure of the first fluid and exerted on the fluid in the second reservoir to be variable. This may be achieved by varying the volume of the first reservoir, for example by means of a portion of the reservoir wall being movable, possibly through a screw or ratchet mechanism. Alternatively, a supplementary reservoir can be provided in communication with the first reservoir, the supplementary reservoir being variable in volume.

Means may be provided, preferably automatically-actuable, for varying the volume of the first reservoir in response to variations in ambient conditions or variations in the parameters or characteristics, such as viscosity, of the first and/or second fluid.

Preferably, means are provided for feeding periodically to the second reservoir aliquots of uniform volume of the fluid to be delivered.

Preferably also, the second reservoir has an inlet in communication with a source of fluid to be delivered. The inlet may be in communication with the source of fluid to be delivered through a one-way valve which prevents return flow of fluid from the reservoir to the source.

In one embodiment of the invention the first fluid is contained in a closed flow loop which includes the first reservoir, the first reservoir being in communication in the loop with a periodically-actuable fluid feed device for providing the first fluid to the first reservoir.

Means can be provided for determining the rate of flow of the second fluid through the fluid flow restricting means. Such rate-determining means may comprise for example a calibration chart for defining the flow rate against such parameters as pressure, temperature, nature of the first and second reservoirs and nature of the fluid flow restricting means.

The fluid flow from the second reservoir may be non-linear with respect to pressure; for example where the reservoirs are syringes, at low pressure much of the force generated may be used in overcoming the inertia or friction of the plunger in the syringe, whereas at high pressure most of the force generated will be available to drive the fluid from the syringe through the fluid flow restricting means. As an example of this, if the pressure of the first fluid is 1 bar, it may require a force deriving from 0.9 bar to move the plungers along the syringes, leaving a net effective pressure of 0.1 bar for driving the second fluid through the flow restricting means. If on the other hand the pressure of the first fluid is 2 bar, the net effective pressure will be 1.1 bar. An increase of a factor of 2 in the pressure of the first fluid therefore produces an increase of a factor of 11 in the effective force for driving the second fluid through the flow restricting means.

It has been found that for a given flow restricting means in the form of fine-bore tubing the flow rate through it is directly proportional to the net pressure induced in the second fluid.

The rate-determining means may be incorporated into software for controlling the supply of pressure to the first fluid.

Embodiments of the present invention will now be described by way of example with reference to the accompanying drawings, in which:
Figure 1(a) is a perspective view of a first embodiment of apparatus of this invention.
Figure 1(b) is a longitudinal cross-sectional view through the central connector 5 of Figure 1(a);
Figure 2(a), (b) and (c) are respectively a front, side and rear perspective view of the apparatus of Figure 1 disposed in a housing;
Figure 3 (a) is a perspective view of a second embodiment of the invention;
Figure 3 (b) is an end view of the central connector 15 of Figure 3 (a);
Figure 3 (c) is a longitudinal cross-sectional view through an upper portion of the connector 15;
Figure 4 (a), (b) and (c) correspond to Figure 3(a), (b) and (c) respectively for a third embodiment of the invention, Figure 4 (a) being an exploded view;
Figure 5 (a) is a perspective view of a fourth embodiment of the invention;
Figure 5 (b) are perspective views of alternative reservoirs to reservoir 34 of Figure 5 (a);
Figure 6 (a), (b) and (c) are respectively a front, back and side cross-sectional view of a fifth embodiment of the invention;
Figure 6 (d) is a schematic view of the apparatus of Figure 6 (a);
Figure 7 is a schematic cross-sectional view through a first reservoir of an embodiment of the invention;
Figure 8 is a schematic view of a fifth embodiment of the invention; and
Figure 9 is a schematic view of a sixth embodiment of the invention.

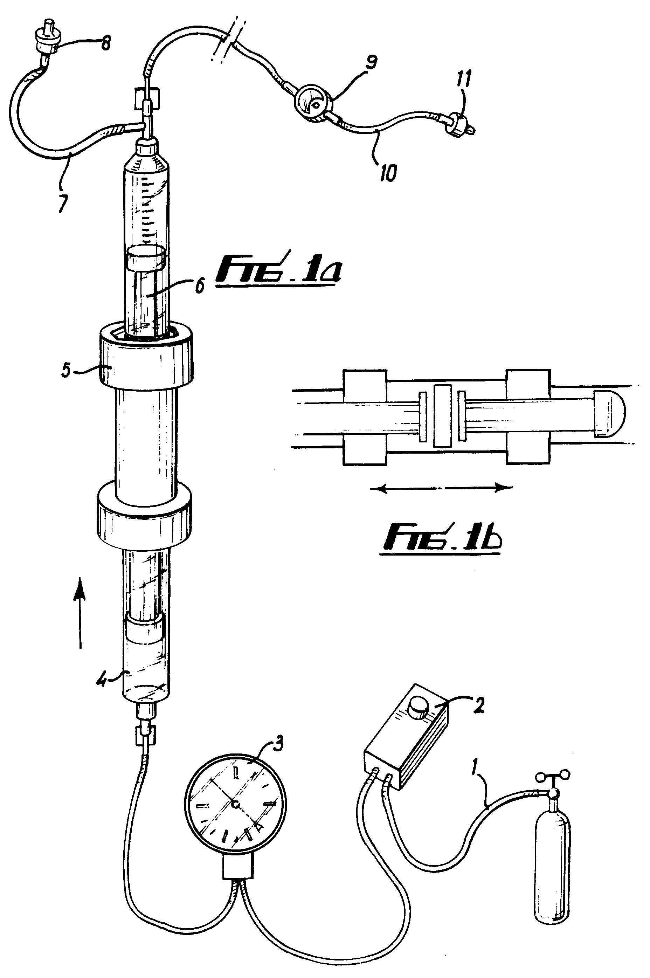

In Figure 1:

1 represents an incoming gas supply line; 2 represents a pressure control valve; 3 represents a pressure gauge; 4 represents a gas-filled syringe; 5 represents a connector having a bayonet fitting to receive a drug-containing syringe. The gas-filled syringe is held in the connector 5 either using a bayonet fitting or a screw mechanism; 6 represents the drug-containing syringe; 7 represents tubing from a T piece which facilitates filling of the drug-containing syringe; 8 represents a one-way valve with a luer lock fitting which allows filling of the drug-containing syringe; 9 represents extension tubing with a filter which connects the drug-containing syringe with the resistance tubing; 10 represents the resistance tubing; 11 represents a luer lock fitting which connects directly to the patient's intravenous cannula.

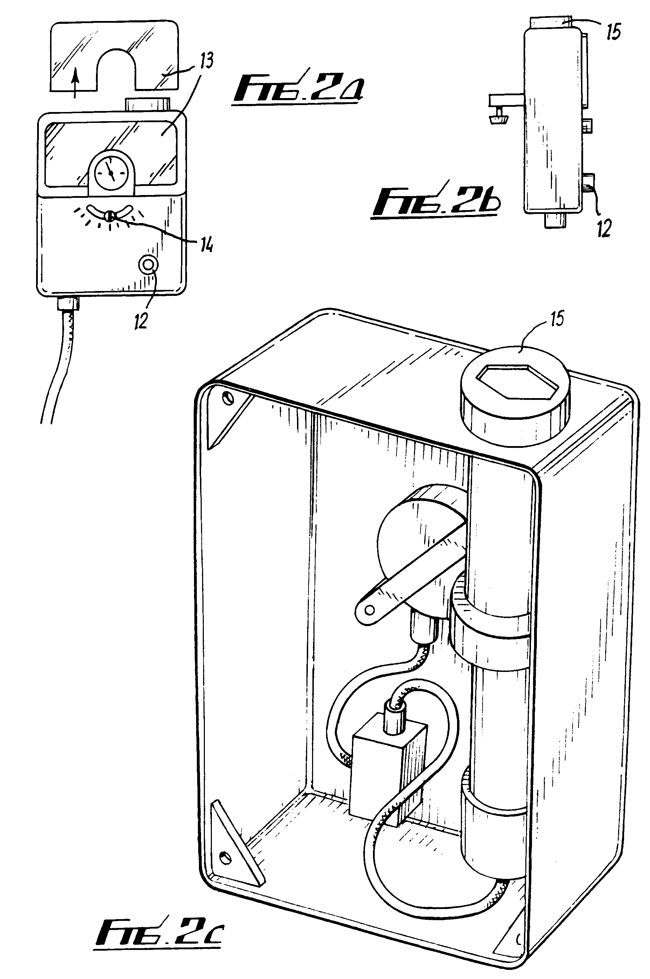

In Figure 2 the components of the apparatus have been arranged in a housing:

12 represents a knob which allows variation of the pressure control valve; 13 represents a chart which can be inserted to represent a specific flow rate for a specific fluid with a known viscosity at a known temperature with pressure exerted against a known flow control tube; 14 represents a knob that rotates the pressure gauge while the flow control chart remains fixed. This rotation of the pressure gauge allows adjustments to be made for variations in temperature. In routine clinical use the operating temperature is set at 22°. Movement of this knob to the left or right allows the calibration to be adjusted by moving the pressure gauge; 15 represents a bayonet fitting suitable for insertion of the drug-containing syringe.

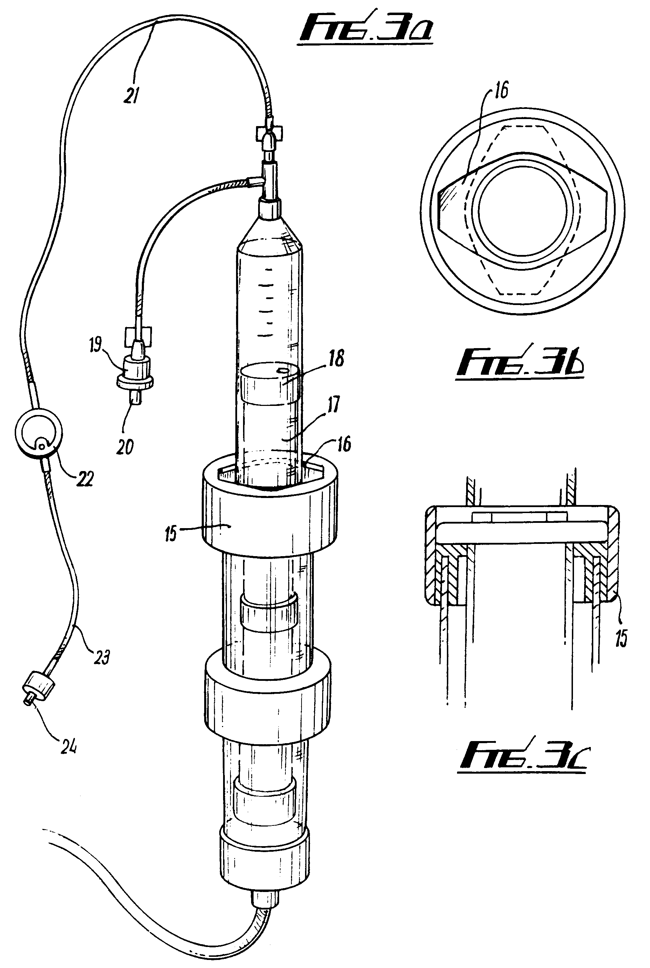

In Figure 3 a standard syringe has been replaced with a modified syringe. In this case a syringe barrel and a plunger can be attached to the bayonet fitting 15. A circular plastic rod with a formed end suitable to fit onto a gasket of the syringe protrudes through the bayonet fitting. The gas-driven system therefore exerts pressure directly on to the drug-containing syringe gasket through this longitudinally moving rod:

16 represents a recess on the bayonet fitting that the syringe wings clip into; 17 represents the circular plastic rod with the formed end suitable to fit onto the syringe gasket; 18 represents the syringe gasket; 19 represents a refill port that allows backfilling of the syringe; 20 represents a one way valve to facilitate backfilling without the use of a tap; 21 represents extension tubing to convey fluid from the syringe to the patient; 22 represents an air and bacteria removing filter; 23 represents fine bore tubing to control the rate of flow; 24 represents a male luer lock fitting.

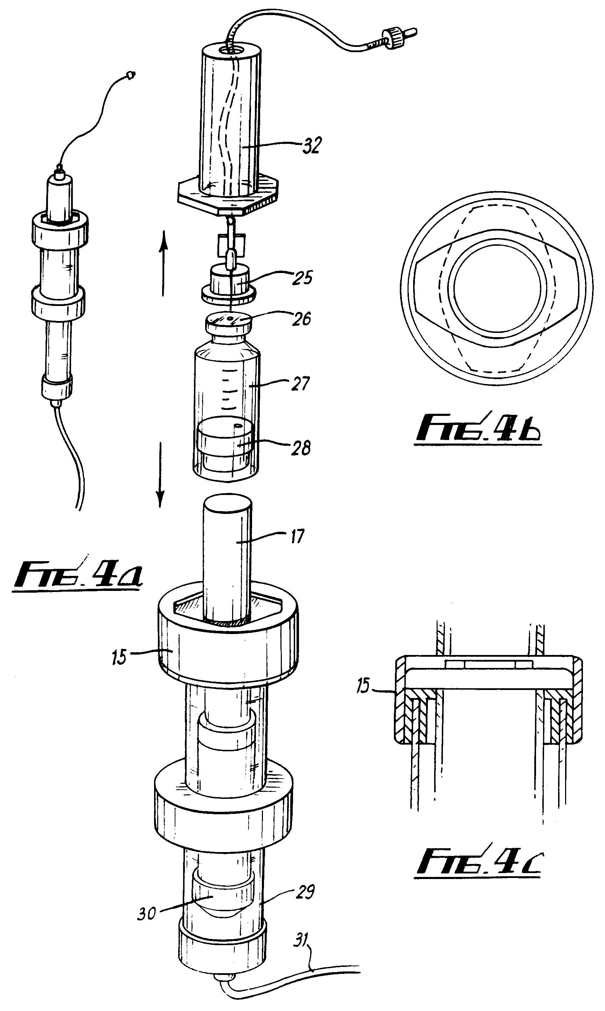

In Figure 4 the modified syringe of Figure 3 has been replaced with a modified glass ampoule. This modified glass ampoule is covered by an outer plastic casing to prevent shattering of glass if excessive pressure is used. The pressure is transmitted to the gasket again by a longitudinally moving rod with a specially formed end to fit the gasket;

25 represents a cover for the glass ampoule with a perforating needle to go through a rubber membrane 26 at the end of the glass ampoule; 27 represents the glass ampoule; 28 represents the rubber gasket of the glass ampoule; 29 represents the syringe containing compressed air; 30 represents the gasket in the syringe containing compressed air which is attached to the longitudinally moving rod which transmits the pressure to the ampoule gasket; 31 represents the compressed air tubing to the variable pressure source; 32 represents an outer plastic casing which provides a protective cover for a glass ampoule when significant pressure is placed on the glass ampoule.

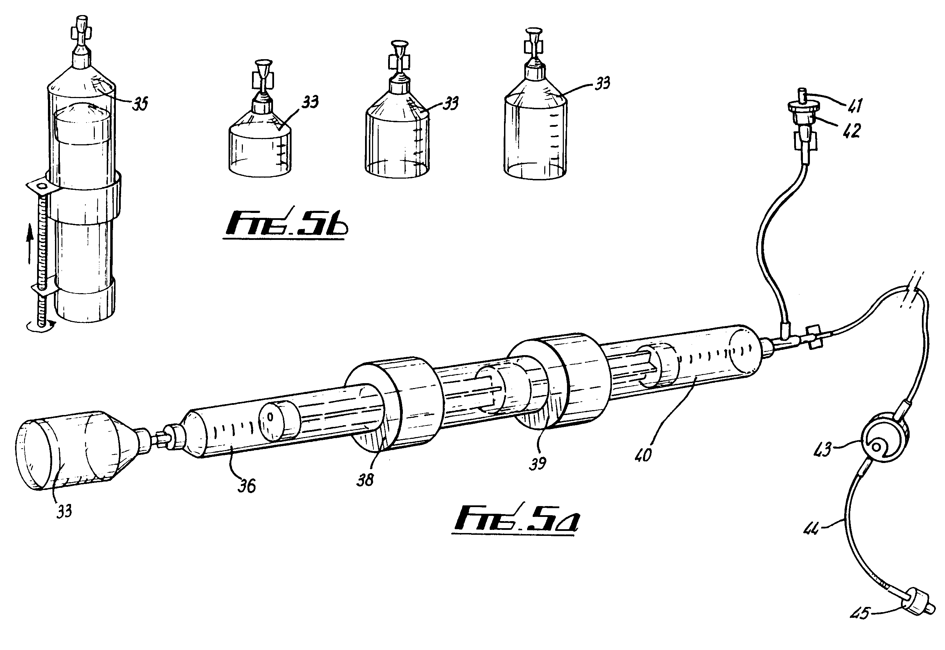

Figure 5 shows four alternative reservoirs 33 and 35. Reservoirs 33 are each fixed though different size reservoirs and 35 represents an adjustable size reservoir.

In Figure 5, 36 represents a syringe connected to one of the reservoirs 33. Prior to use the syringe is used to compress air into the reservoir 33 and is then inserted into a bayonet fitting 38. A bayonet fitting 39 holds in place the drug-containing syringe 40. The syringe 36 has a pressure inversely proportional to the size of the reservoir. The smaller reservoir 33 therefore produces a higher pressure than the larger reservoir. In the case of the reservoir 35 the size can be varied and the pressure can therefore be varied accordingly:

41 represents a refill port that allows backfilling of the syringe; 42 represents a one-way valve to facilitate backfilling without the use of a tap; 43 represents an air and bacteria removing filter; 44 represents fine bore tubing to control the rate of flow; 45 represents a male luer lock fitting.

This family of syringe-driven pumps allows for extremely simple pumps to be designed where the pressure can be changed by charging a gas-driven reservoir with a fixed volume. Injecting a fixed volume of air into that reservoir gives a fixed pressure head. As an alternative system a more sophisticated pump can be used where it is possible to vary the pressure and control the pressure throughout the full movement of the syringe. This range of pumps gives significant benefits over electronic pumps.

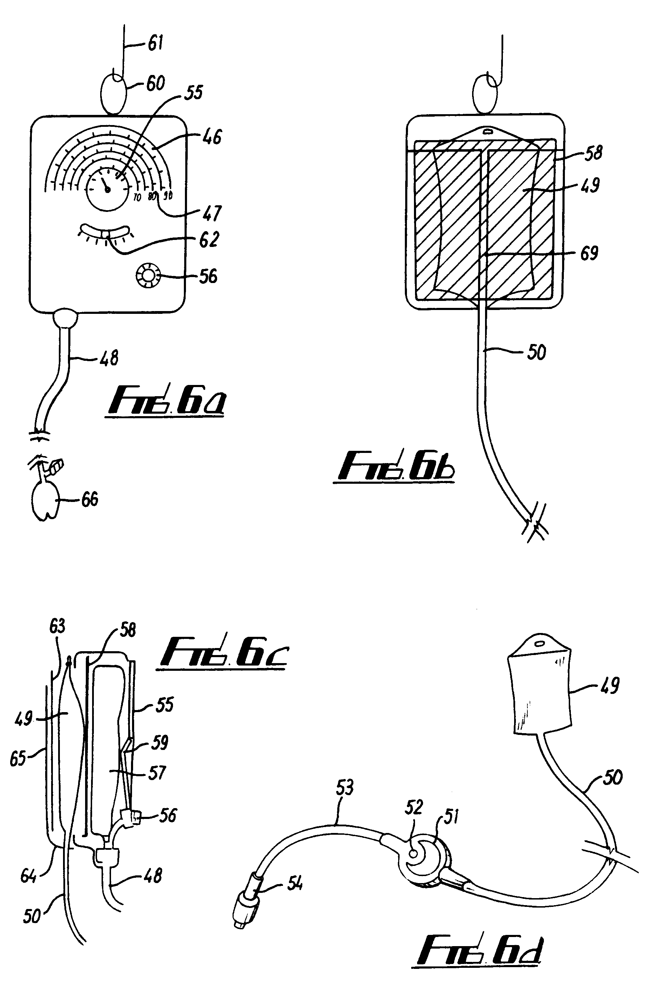

In Figure 6 a further embodiment of the invention is demonstrated. In this embodiment the variable gas-driven pressure is calibrated so that a balloon 57 presses on a moveable segment 58 which causes pressure on a minibag 49. The minibag then delivers fluid through its tubing 50. The flow rate is controlled by flow control tubing 53 which has a relatively narrow lumen:

46 represents a calibrated flow rate diagram showing flow in mls/hr and also in mgs/kg/hr; 47 represents a segment of the diagram referring to calibration for weight so that the flow can be calibrated in mgs/kg/hr with a set pressure against a set resistor; 48 represents a gas supply to a pump; 49 represents a flexible bag containing drug or fluid; 50 represents tubing coming from the flexible bag or drug container (minibag) 49;
51 represents a bacterial filter in the line; 52 represents an air removing filter in the line; 53 represents a segment of tubing with a narrow lumen which controls the rate of flow from the minibag 49; 54 represents a male luer lock fitting which allows the bag 49 to be connected to the patient; 55 represents a pressure gauge; 56 represents a valve which can be turned to control the pressure level; 57 represents an elastic bag which can be inflated to produce pressure on a mobile plate; 58 represents the mobile plate; 59 represents tubing between the pressure control valve 56 and the pressure gauge 55; 60 represents a ring which allows suspension of the device from a drip pole;

61 represents a segment of the drip pole; 62 represents a knob for moving the pressure gauge around its axis so that adjustments can be made from variations in temperature. These are made by adjusting the orientation of the pressure gauge in relation to the flow control chart above it; 63 represents a plate that can slide into place after the minibag 49 has been inserted; 64 represents an opening which allows the tubing of the minibag to be inserted easily. The plate 63 is then inserted once the minibag is in place. It is important that the plate 63 and a wall 65 of the container are made of a clear transparent material so that the minibag can be viewed at all times; 66 represents a balloon with a one way valve which allows air to be pumped into the pressure device so as to give a specific rate of flow from the minibag 49.

This arrangement of the pressure gauge has orientation to the calibrated chart which can be varied. This allows accurate flow rates to be expressed in mgs/kg/min with variations in temperature taken into consideration. It would be normal to provide a chart for a drug of a specific viscosity as this allows packaging of a drug of a specific viscosity in the minibag with a known resistor. A combination of this variable gas driven system together with the temperature compensating technique allows this style of pump to be designed for a specific drug with a specific resistor. The drug can therefore be packaged in this type of container.

These embodiments of the invention include syringe pumps which can be powered by gas. This allows the development of extremely low cost gas powered pumps. This range includes some that allow the flow to be turned up or down by increasing the gas pressure driving a syringe while others allow variation in pressure by varying the volume of a reservoir or alternatively selecting reservoirs of an appropriate volume so that an appropriate pressure will be generated when a fixed volume of air is injected into the reservoir.

Each of these pump designs involves a syringe which is filled with a fluid, for example a gas, at a specific pressure. The fluid then provides pressure on a plunger which transmits force longitudinally to a syringe which is placed back-to-back. The thumb piece of the syringe containing air then presses directly on the thumb piece of the syringe containing drug. The wings of both syringes are held within an appropriately designed housing that essentially provides a bayonet fitting for the wings of each syringe. This provides a system where the pressure in the gas-driven syringe is transmitted directly to the fluid- or drug-containing syringe with no risk of air leaks from the gas-driven syringe to the drug-containing syringe.

The drug- or other fluid-containing syringe can be a standard plastic syringe. Alternatively it may be in the form of a glass ampoule with a moveable plunger (Figure 4). In this embodiment the glass ampoule is usually covered with a protective cover that fits within the bayonet fitting and the gas-driven syringe has a member that inserts into the rubber plunger of the ampoule or into the rubber plunger of a modified syringe. In the case of a modified syringe the syringe barrel and rubber gasket form the drug container. This modified syringe can be inserted into the bayonet fitting and the gasket will be driven by the longitudinal member that transmits pressure from the gasket of the gas-driven syringe.

When using this technology the fluid-containing syringe can be filled before use and then loaded into the device. An alternative method of filling the syringe allows filling from the distal end of the syringe by use of a T-piece with a one way valve allowing direct injection into the syringe. The direct injection into the syringe is facilitated by the fact that the syringe pumps against relatively high resistance tubing in order to provide a constant infusion.

Conventional electronic pumps have a very wide range so that the pump can run from 0mls per hour to 1,000mls per hour. The air-driven pump system of these embodiments of the invention can effectively work between 0.5 and 4 bars pressure with commercial syringes. While it may be possible to operate between 0 and 0.5 bars pressure the accuracy of the pump in this low pressure range decreases because of the variations in resistance caused by syringe gaskets. For practical purposes therefore the pump will usually be set at a standard operating level of 1 bar initially where it can be increased four fold but not a thousand fold as could occur with an electronic pump. This limitation provides an element of safety in some situations.

The pumps systems of these embodiments can be calibrated for a specific drug with a specific viscosity. This allows the pump to be calibrated to give a specific drug in ml/kg/hr. This will ensure in many situations that the pump can only be used for the drug that it has been manufactured and designed for.

Some conventional electronic pumps are at risk of sudden downloading of drugs accidentally from their syringes, but in the apparatus of the present embodiments of the invention the resistance tubing prevents sudden downloading of drugs. Further, the pressure gradient across the high resistance tubing will usually be sufficient to prevent syphoning.

The present embodiments of the apparatus do not depend on electronics and the patient is therefore protected from microelectric shocks. The pump can be operated from a conventional compressed air source, such as compressed air bottles. In order to maximise safety in the pump circuit it is essential to have a blow-off valve if connected to a compressed air bottle. The standard operating pressures in most theatres and hospitals will have a maximum of 4 bar and it is easy to produce plastic fittings safe at least to 4 bars pressure while the valve is safe at approximately 6 bars pressure which is usually adequate to protect the pump. This gives protection if connected directly to the full pressure of a gas bottle.

An alternative form of compressed air is the use of a standard foot pump or alternatively a syringe to inject air through a one-way valve into a reservoir connected to the driving syringe.

One of the disadvantages of electronic syringe pumps is the difficulty of providing a continuous infusion at the time of changing syringes. This often leads to an absence of flow for one or two minutes while the syringe is being changed. In the case of drugs with a half life of one or two minutes this may predispose to significant physiological changes that occur for the patient.

The syringes of these embodiments of the invention provide a system where the syringe can be refilled without interruption of flow. The filling of the syringe can be performed through a one-way valve. As the syringe fills the plunger is pushed back. In the case of the syringe pump driven with compressed air at a preset level the pressure in the driving syringe is maintained constant at all times by a valve that controls this pressure level. The filling of the syringe is therefore not associated with significant increases in pressure in the drug-filled syringe as there are minimal pressure changes in the drug-filled syringe and the actual flow continues to be constant. It is therefore possible to refill the syringe while maintaining a constant flow. This provides a significant advantage when using vasoactive drugs.

The apparatus of these embodiments use precalibrated fine-bore resistance tubing controlling the rate of flow of fluid from the drug-containing syringe.

The control of flow through fine-bore tubing is viscosity dependent. This means that the calibrations on the pump need to be set for a specific viscosity. It is important that whoever uses the pump should select a calibration system appropriate for the appropriate viscosity.

The system is temperature dependent and it is therefore necessary to adjust the calibration chart around the pressure manometer against a specific temperature. In Figure 1 the pressure gauge 3 is shown as a circular dial with specific pressure readings consistent with specific flow rates. In the design of instrument shown in Figure 1 the dial can be moved a few degrees to the right or to the left so that the relationship between the dial and the chart shows flow rate changes. This allows for a correction in flow rate that occurs with temperature.

In general a 2.5% increase in flow rate will be noted for each 1 degree centigrade rise in temperature. In practice the pressure-reading dial can be turned to a position that compensates for this variation in temperature.

With the embodiment illustrated in Figure 5 a refill port 41 is designed to allow backfilling of a syringe 40 and a filter 43 is placed in line between the syringe 40 and the flow control tubing 44. This filter 43 eliminates air bubbles entering the flow control tubing 44 and prevents bacteria reaching the patient. This positioning of this filter 43 is integral to getting maximum function from the flow control tube 44 (air bubbles tend to block the tube). The positioning of the filter 43 is also critical in terms of protecting the patient from any possible contamination that might occur with repeated refilling of the syringe 40.

In these two-syringe systems one of the syringes can have a volume which is variable and selectable by means of a ratchet or screw mechanism. The other syringe can have a plunger that moves in response to the pressure in its chamber.

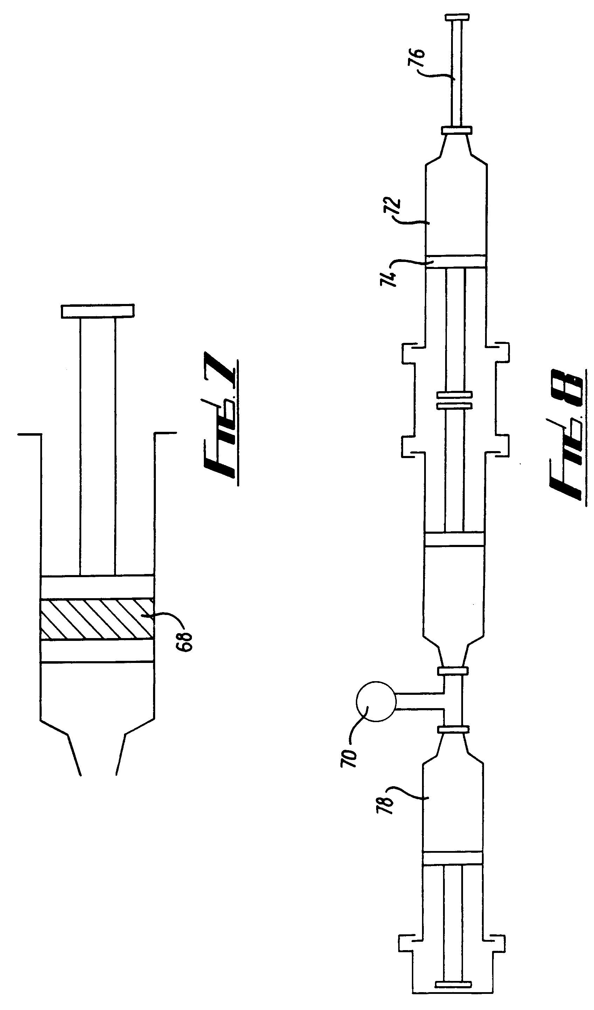

In the embodiment the pressure driving syringe can be intermittently and selectively attached to any of a series of reservoirs. The pressure in the pressure driving syringe will be inversely proportional to the size of the reservoir that it is connected to. When connected to a small reservoir and depressed fully, the pressure will be extremely high, while when connected to a large reservoir the pressure will be low. By appropriate labelling of each reservoir, it is possible to have a known pressure within the syringe providing no leaks occur within the system. Leaks may be eliminated by a hydraulic seal gasket 68 (see Figure 7).

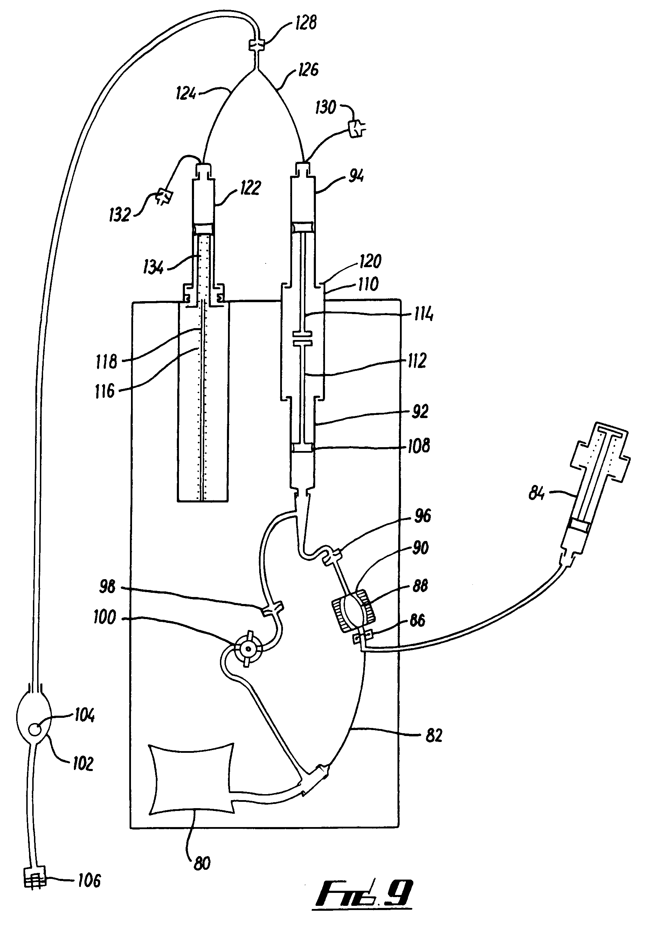

An alternative embodiment involves a system of connecting the pressure driving syringe to a variable reservoir, as for example in Figure 8. If the size of the reservoir is varied, the pressure within the pressure driving syringe varies itself. With this embodiment the pressure within the pressure driving syringe can be varied during use of an infusion pump. The reservoir can be calibrated against pressure, flow or mg/kg/minute of drug being infused.

In simple embodiments of the invention the pressure can be calibrated on the series of reservoirs, or against a known position on a variable reservoir syringe. With some embodiments, a T-piece on the line connecting the two syringes can allow a pressure gauge 70 to be integrated into the circuit (see Figure 8). This pressure gauge 70 can be connected electronically to an appropriate computer or programme. This programme can control flow in response to pressure, temperature, viscosity, drug concentration and weight of the patient. In this circumstance the computer can express the number of milligrams per kilogram per hour with corrections for viscosity and temperature variations built in to the formula.

The apparatus can therefore include a series of reservoirs or alternatively a variable reservoir. A tube connects the variable reservoir to a pressure driving syringe fixed in position within a syringe holding device so that the thumb pieces on the barrel push firmly on the distal end of the housing, and so that the plunger connects directly with the plunger of the drug-containing syringe with a longitudinal connection between both of these. The pressure from the pressure driving syringe therefore is transmitted as direct pressure on the plunger of the drug-containing syringe, or other container.

In Figure 8, the drug-containing syringe 72 abuts against the proximal end of the housing and has pressure directly transmitted to its rubber plunger 74. Its pressure is transmitted from the rubber plunger onto the fluid contained with the syringe, which is delivered slowly through finely calibrated flow control tubing 76. This flow control tubing 76 then delivers fluid at a predetermined rate to the patient.

In the event that the rate needs to be increased, the pressure is increased by an appropriate amount. Doubling the pressure will directly double the flow rate. This can be achieved easily by decreasing the space in the variable reservoir syringe 78, or alternatively choosing a precalibrated pressure head at the appropriate level.

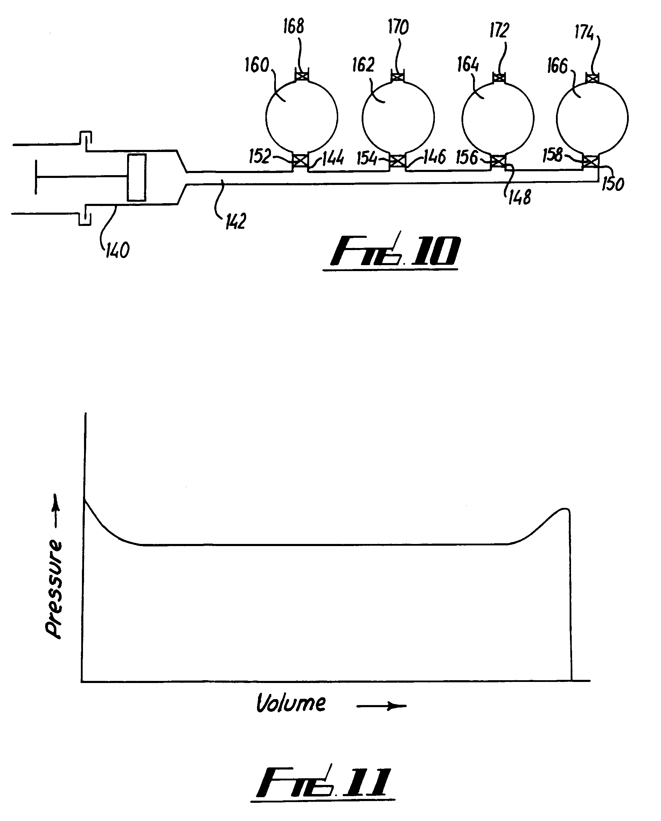

In Figure 9 there is provided apparatus for delivering fluid on a continuous basis from a first reservoir such as a syringe or elastomer driven container and fluid as required by the patient from a second reservoir such as a syringe. Each reservoir can be independently examined to confirm how much drug or fluid has been delivered to the patient.

The energy to the constant-infusion syringe can be delivered by a spring-driven syringe or elastomer. The rate of egress of fluid is controlled by tubing with a fine lumen sufficient to provide a resistance to flow at a present rate.

The patient controlled circuit is hydraulically controlled by an internal circuit that is reused and an external circuit that controls the delivery of energy of fluid from the internal circuit.

The internal circuit provides a time delay mechanism and an energy-containing reservoir with a limited energy store. The limited energy store delivers pressure to a longitudinal syringe or piston which delivers pressure to the patient controlled syringe or reservoir.

The patient controlled syringe or reservoir then delivers pressurised fluid to a flow control resistor that controls the rate of delivery of fluid from the patient controlled syringe. This resistor to flow in Figure 9 is fine lumen tubing but can alternatively be any form of resistance such as a fine aperture in a membrane or a filter.

In the preferred embodiment each independent syringe can be filled through a one-way valve which allows refilling of the syringe. A tap mechanism allows the internal circuit to be opened to allow fluid to return to a flexible reservoir when the patient controlled reservoir or syringe is being refilled.

80 represents a reservoir in the form of a flexible bag within the reusable internal circuit. This flexible bag contains fluid and acts as a flexible reservoir for the internal hydraulic circuit.

82 represents the fine bore tubing which provides a restriction to flow of fluid between the reservoir 80 and an aspirating syringe 84. The fine bore tubing 82 restricts the flow of fluid and controls the rate of filling of the aspirating syringe 84.

The aspirating syringe 84 is spring loaded and aspirates fluid from the internal circuit. The rate at which fluid is aspirated is controlled by the fine bore tubing 82. It should be noted that one-way valve 86 prevents entry of fluid from a balloon energy-containing reservoir 88. The aspirating syringe 84 has a spring contained within a housing which provides a push-button appearance and controls the length of longitudinal movement of the syringe. The housing therefore controls the filling volume of the syringe. The aspirating syringe 84 therefore has an ability to fill to a fixed volume at a fixed rate.

90 represents a strong housing shaped around the elastomeric balloon 88. The housing 90 controls the volume to which the balloon 88 can be filled. The housing 90 is therefore shaped internally in the same shape as the balloon 88 when filled. The housing 90 can limit the volume in the energy-containing balloon 88 to a volume similar to the volume contained in the aspirating syringe 84. In this way the housing 90 can control the number of boluses of fluid in the energy reservoir of the elastomeric balloon 88 at any one time.

88 represents the elastomeric balloon with relatively thick walls. This elastomeric balloon can generate quite high pressures which can be transferred on to a longitudinal driving syringe 92. The elastomeric balloon 88 is filled when the aspirating syringe 84 is depressed by the patient or nurse. The balloon 88 then contains a fixed volume of fluid with a pressure generated by the walls of the elastomer. The pressure is transferred to the driving and patient controlled syringes 92, 94.

96 represents a one-way valve which prevents the return of fluid from the driving syringe 92 to the elastomeric balloon 88.

98 represents a one-way valve which prevents the return of fluid from the reservoir fluid bag 80 to the driving syringe.

100 represents a spring-loaded tap which is usually in the closed position during use preventing any flow of fluid from the driving syringe 92 to the flexible reservoir bag 80. During refilling of the patient controlled syringe 94 the tap 100 is opened to allow fluid to move from the driving syringe 92 direct to the flexible reservoir bag 80.

102 represents a filter to protect the patient from any bacterial contamination of the fluid within the external patient circuit.

104 represents a hydrophobic air removing filter to protect the patient from any air bubbles within the circuit.

106 represents a male luer lock fitting to connect the infusion device to a standard intravenous line.

The driving syringe 92 is held in a longitudinal tube. The driving syringe 92 receives pressure from the elastomeric balloon 88 when it is filled with fluid. The pressure is transmitted to a rubber seal 108 by the fluid within the driving syringe 92. The pressure in the driving syringe 92 becomes equal to the pressure in the elastomeric balloon 88. This pressure is transmitted onto the rubber seal and transferred along the longitudinal member to the patient controlled syringe 94. This patient controlled syringe 94 is held in a bayonet fitting 110 with the plunger thumb or piece 114 of the patient controlled syringe 94 abutting directly against the plunger or thumb piece 112 of the driving syringe 92. The pressure within the driving syringe 92 is therefore transferred to the patient controlled syringe 94 so that the pressure in the fluid compartment of the patient controlled syringe 94 is a similar pressure to the pressure in the driving syringe 92. The difference in pressure between the two syringes relates to the amount of energy taken up by the resistance in the plungers of the driving syringe 92 and the patient controlled syringe 94.

In ideal circumstances this resistance is close to zero.

116 represents an energy-containing spring on a constant infusion syringe 22.

118 represents a stem which prevents kinking of the long spring 116 of the constant infusion syringe 122.

120 represents a bayonet fitting which receives the wings of the patient controlled syringe 94. A similar bayonet fitting is used to hold the wings of the spring-loaded constant infusion syringe 122.

124 represents fine bore tubing which controls the egress of fluid from the constant infusion syringe 122.

126 represents fine bore tubing which controls the rate at which energy or fluid is delivered from the patient controlled syringe 94.

128 represents a high pressure valve which is designed to prevent any risk of syphoning of fluid from either syringe 94, 122. This high pressure valve 128 has an opening pressure for the valve which is significantly higher than could occur between the top of the device and the patient at any time. This anti-siphon valve 128 simply protects the patient from the syphoning of fluid.

130 represents a one-way valve designed to allow injection of fluid directly into the patient controlled syringe 94. When this is being performed it is important that the tap 100 is open so that as the patient controlled syringe 94 fills, and as fluid is pushed from the driving syringe 92 that fluid returns directly to the flexible reservoir bag 80.

132 represents a one-way port into the continuous infusion syringe 122.

134 represents a housing which contains the spring of the constant infusion syringe 122 and fits inside the continuous infusion syringe. It is designed in such a way that it can move longitudinally the full distance of the constant infusion syringe 122 and push the rubber plunger as far as the end of the patient controlled syringe 94. The continuous infusion syringe 122 can move up and down its longitudinal compartment. This housing 134 provides a system whereby the spring can effectively travel virtually the full length of the continuous infusion syringe 122.

This embodiment provides the basic principles of an internal hydraulic circuit with a time delay switch and an energy containing reservoir with a limited volume. The external circuit as described provides a rate controlling mechanism for transfer of energy from this internal circuit. The rate controlling mechanism plus the anti-siphon valve provides the patient with protection. The patient is further offered protection by the internal circuit and its time delay mechanism as well as the limited quantity of energy which can be stored in the internal circuit at any time.

This device therefore provides a background infusion and intermittent boluses as required. Many applications with medicine require a background infusion and a maximum infusion rate, and this device can be applied to such situations in medicine and also to other industrial applications.

Modifications and improvements may be incorporated without departing from the scope of the invention.

For example, Figure 10 is a schematic diagram of a part of fluid delivery apparatus in accordance with a further embodiment of the invention in which a first syringe 140 is powered by its connection to an inlet 142 which has along it four branch inlets 144, 146, 148, 150. Each of the branch inlets is connected to the main inlet 142 through a valve 152, 154, 156, 158 each of which is selectively actuable independently of the others. The branch inlets receive compressed gas from respective balloon reservoirs 160, 162, 164, 166 which have different gas pressure levels. Each of the balloon reservoirs has a one way valve 168, 170, 172, 174 for charging with gas.

In this modification, the volume of each balloon reservoir 160, 162, 164, 166 is greater than the volume of the syringe 140, to the effect that the pressure is constant for 90% of the reservoir's volume, as shown in the graph of Figure 11.

## Claims

1. Apparatus for delivery of fluid to a patient, the apparatus comprising a first reservoir (4, 29, 57, 92) for containing a pressurized first fluid, a second reservoir (6, 27, 49) for containing the fluid to be delivered and having an outlet therefor, means (5, 17, 58) between the first and second reservoirs for transferring a force produced by the pressure of the first fluid to the fluid of the second reservoir (6, 27, 49), characterised in that a tube (10, 53, 126) having a narrow lumen is disposed between the outlet of the second reservoir (6, 27, 49) and the patient, and where the rate of fluid delivery to the patient is determined by the diameter of the lumen of the tube (10, 53, 126).

2. Apparatus as claimed in Claim 1, wherein means (1, 12, 13, 14) are provided for varying the pressure of the first fluid in the first reservoir (4, 29, 57, 92).

3. Apparatus as claimed in Claim 1 or 2, wherein the first reservoir has an inlet for receiving said first fluid, the inlet being in communication with a pressurised source of said first fluid.

4. Apparatus as claimed in Claim 1 2 or 3, wherein the second reservoir (6, 27, 49) comprises a cylinder and said means for transferring a force comprises a piston (17) movable therealong.

5. Apparatus as claimed in any preceding Claim, wherein the first reservoir (4, 29, 57) comprises a cylinder and said means for transferring a force comprises a piston (17) movable therealong.

6. Apparatus as claimed in Claim 5 when dependent on Claim 4, wherein said cylinders are connected together in tandem.

7. Apparatus as claimed in Claim 6, wherein the cylinders are connected together through a connector member (5) which engages each of the cylinders through a bayonet-type fitting.

8. Apparatus as claimed in any one of the preceding Claims, wherein the first reservoir (4, 29, 57, 92) is variable in volume.

9. Apparatus as claimed in Claim 8, wherein the volume of the first reservoir is variable by means of a screw or ratchet mechanism.

10. Apparatus as claimed in Claim 9, wherein a supplementary reservoir (35) is in communication with the first reservoir, the supplementary reservoir (35) having a variable volume.

11. Apparatus as claimed in Claim 8, 9 or 10, wherein means are provided for varying the volume of the first reservoir in response to variations in ambient conditions.

12. Apparatus as claimed in any one of Claims 8 to 11, wherein means are provided for varying the volume of the first reservoir in response to one or more parameters of the fluid to be delivered.

13. Apparatus as claimed in any one of the preceding Claims, wherein means (7, 19, 20, 41, 42, 130) are provided for feeding periodically to the second reservoir (6, 27, 49) aliquots of uniform volume of the fluid to be delivered.

14. Apparatus as claimed in any one of the preceding Claims, wherein the second reservoir (6, 27, 49) has an inlet (7, 19, 20, 21, 41, 42, 130) in communication with a source of fluid to be delivered.

15. Apparatus as claimed in Claim 14, wherein the inlet (7, 19, 20, 21, 41, 42, 130) is in communication with the source of fluid to be delivered through a one-way valve which prevents return flow of fluid from the reservoir to the source.

16. Apparatus as claimed in any one of the preceding Claims, wherein a filter (9, 22, 43, 51, 102) for removal of bacteria is provided downstream of the second reservoir (6, 27, 49).

17. Apparatus as claimed in any one of the preceding Claims, wherein means (22, 43, 52, 104) for removing air from the fluid being delivered is located between the second reservoir (6, 27, 49) and the tube (10, 53, 126).

18. Apparatus as claimed in any one of the preceding Claims, wherein a pressure gauge (3) is provided in communication with the first reservoir (4, 29, 57, 92).

19. Apparatus as claimed in any one of the preceding Claims, wherein the first fluid is contained in a closed flow loop which includes the first reservoir (4, 29, 57, 92), the first reservoir being in communication in the loop with a periodically-actuable fluid feed device (84) for providing the first fluid to the first reservoir (4, 29, 37, 92).

20. Apparatus as claimed in any one of the preceding Claims, wherein means are provided for determining the rate of flow of the second fluid through the tube (10, 53, 126).

## Patentansprüche

1. Vorrichtung zur Abgabe von Flüssigkeit an einen Patienten, wobei die Vorrichtung aus einem ersten Behälter (4, 29, 57, 92) zum Beinhalten einer ersten unter Druck gesetzten Flüssigkeit besteht, einem zweiten Behälter (6, 27, 49) zum Beinhalten der abzugebenden Flüssigkeit, der dafür einen Auslaß aufweist, einer Einrichtung (5, 17, 58) zwischen dem ersten und dem zweiten Behälter zum Übertragen einer von dem Druck der ersten Flüssigkeit erzeugten Kraft auf die Flüssigkeit des zweiten Behälters (6, 27, 49), dadurch gekennzeichnet, daß ein Schlauch (10, 53, 126) mit engem Lumen zwischen dem Auslaß des zweiten Behälters (6, 27, 49) und dem Patienten angeordnet wird und daß die Menge der Flüssigkeitsabgabe an den Patienten durch den Durchmesser des Lumens des Schlauchs (10, 53, 126) bestimmt wird.

2. Vorrichtung nach Anspruch 1, wobei Einrichtungen (1, 12, 13, 14) zum Variieren des Drucks der ersten Flüssigkeit in dem ersten Behälter (4, 29, 57, 92) bereitgestellt werden.

3. Vorrichtung nach Anspruch 1 oder 2, wobei der erste Behälter einen Eingang zum Aufnehmen der ersten Flüssigkeit aufweist, wobei der Eingang mit einer unter Druck gesetzten Quelle der ersten Flüssigkeit in Verbindung steht.

4. Vorrichtung nach Anspruch 1, 2 oder 3, wobei der zweite Behälter (6, 27, 49) einen Zylinder umfaßt und die Einrichtung zum Übertragen einer Kraft einen darin entlang bewegbaren Kolben (17) umfaßt.

5. Vorrichtung nach einem der vorhergehenden Ansprüche, wobei der erste Behälter (4, 29, 57) einen Zylinder umfaßt und die Einrichtung zum Übertragen einer Kraft einen darin entlang bewegbaren Kolben (17) umfaßt.

6. Vorrichtung nach Anspruch 5, wenn von Anpruch 4 abhängig, wobei die Zylinder in Tandemanordnung miteinander verbunden sind.

7. Vorrichtung nach Anspruch 6, wobei die Zylinder durch ein Verbindungselement (5), welches jeden der Zylinder durch ein bajonettartiges Anschlußstück anschließt, miteinander verbunden sind.

8. Vorrichtung nach einem der vorhergehenden Ansprüche, wobei der erste Behälter (4, 29, 57, 92) im Volumen veränderlich ist.

9. Vorrichtung nach Anspruch 8, wobei das Volumen des ersten Behälters mittels eines Schraub- oder eines Ratschenmechanismus veränderlich ist.

10. Vorrichtung nach Anspruch 9, wobei ein Zusatzbehälter (35) mit dem ersten Behälter in Verbindung steht, wobei der Zusatzbehälter (35) ein veränderliches Volumen hat.

11. Vorrichtung nach Anspruch 8, 9 oder 10, wobei Einrichtungen zum Variieren des Volumens des ersten Behälters in Abhängigkeit von Veränderungen der Umgebungsbedingungen bereitgestellt werden.

12. Vorrichtung nach einem der Ansprüche 8 bis 11, wobei Einrichtungen zum Variieren des Volumens des ersten Behälters in Abhängigkeit von einem oder mehreren Parametern der abzugebenden Flüssigkeit bereitgestellt werden.

13. Vorrichtung nach einem der vorhergehenden Ansprüche, wobei Einrichtungen (7, 19, 20, 41, 42, 130) zum periodischen Zuführen von Aliquoten eines einheitlichen Volumens der abzugebenden Flüssigkeit in den zweiten Behälter (6, 27, 49) bereitgestellt werden.

14. Vorrichtung nach einem der vorhergehenden Ansprüche, wobei der zweite Behälter (6, 27, 49) einen Eingang (7, 19, 20, 21, 41, 42, 130) aufweist, der mit einer Quelle der abzugebenden Flüssigkeit in Verbindung steht.

15. Vorrichtung nach Anspruch 14, wobei der Eingang (7, 19, 20, 21, 41, 42, 130) durch ein Einwegventil, welches das Zurückfließen von Flüssigkeit von dem Behälter in die Quelle verhindert, mit der Quelle der abzugebenden Flüssigkeit in Verbindung steht.

16. Vorrichtung nach einem der vorhergehenden Ansprüche, wobei ein Filter (9, 22, 43, 51, 102) zum Entfernen von Bakterien stromabwärts des zweiten Behälters (6, 27, 49) bereitgestellt wird.

17. Vorrichtung nach einem der vorhergehenden Ansprüche, wobei eine Einrichtung (22, 43, 52, 104) zum Entfernen von Luft aus der Abgabeflüssigkeit zwischen dem zweiten Behälter (6, 27, 49) und dem Schlauch (10, 53, 126) angeordnet ist.

18. Vorrichtung nach einem der vorhergehenden Ansprüche, wobei ein Druckmesser (3) in Verbindung mit dem ersten Behälter (4, 29, 57, 92) bereitgestellt wird.

19. Vorrichtung nach einem der vorhergehenden Ansprüche, wobei die erste Flüssigkeit in einem geschlossenen Fließkreis beinhaltet ist, welcher den ersten Behälter (4, 29, 57, 92) einschließt, wobei der erste Behälter in dem Kreis mit einer periodisch betätigbaren Flüssigkeitszuführungsvorrichtung (84) zur Bereitstellung der ersten Flüssigkeit an den ersten Behälter (4, 29, 57, 92) in Verbindung steht.

20. Vorrichtung nach einem der vorhergehenden Ansprüche, wobei Einrichtungen zur Bestimmung der Fließmenge der zweiten Flüssigkeit durch den Schlauch (10, 53, 126) bereitgestellt werden.

## Revendications

1. Un appareil pour distribuer du fluide à un patient, l'appareil comprenant un premier réservoir (4, 29, 57, 92) pour contenir un premier fluide sous pression, un second reservoir (6, 27, 49) pour contenir le fluide à distribuer et ayant une sortie à cet usage, un moyen (5, 17, 58) entre le premier et le second réservoir pour transférer une force produite par la pression du premier fluide vers le fluide du second réservoir (6, 27, 49), caractérisé en ce qu'un tube (10, 53, 126) de lumen étroit est placé entre la sortie du second réservoir (6, 27, 49) et le patient, et où la vitesse de distribution du fluide au patient est déterminée par le diamètre du lumen du tube (10, 53, 126).

2. Un appareil selon la Revendication 1, dans lequel des moyens (1, 12, 13, 14) sont fournis pour faire varier la pression du premier fluide dans le premier réservoir (4, 29, 57, 92).

3. Un appareil selon les Revendications 1 ou 2, dans lequel le premier réservoir a une entrée afin de recevoir ledit premier fluide, l'entrée communicant avec une source sous pression dudit premier fluide .

4. Un appareil selon les Revendications 1, 2 ou 3, dans lequel le second réservoir (6, 27, 49) comprend un cylindre et ledit moyen de transfert d'une force comprend un piston (17) mobile sur sa longueur.

5. Un appareil selon l'une des Revendications précédentes, dans lequel le premier réservoir (4, 29, 57) comprend un cylindre et ledit moyen de transfert d'une force comprend un piston (17) mobile sur sa longueur.

6. Un appareil selon la Revendication 5 lorsqu'elle dépend de la Revendication 4, dans lequel lesdits cylindres sont connectés ensemble en tandem.

7. Un appareil selon la Revendication 6, dans lequel les cylindres sont connectés ensemble par l'intermédiaire d'un élément de raccordement (5) qui fait s'engager chacun des cylindres à travers une douille à type baïonnette.

8. Un appareil selon l'une des Revendications précédentes, dans lequel le premier réservoir (4, 29, 57, 92) est de volume variable.

9. Un appareil selon la Revendication 8, dans lequel le volume du premier réservoir est modifiable au moyen d'un mécanisme à vis ou à cliquet.

10. Un appareil selon la Revendication 9, dans lequel un réservoir supplémentaire (35) communique avec le premier réservoir, le réservoir supplémentaire (35) ayant un volume variable.

11. Un appareil selon les Revendications 8, 9 ou 10, dans lequel des moyens sont fournis afin de faire varier le volume du premier réservoir en réponse aux variations dans des conditions ambiantes.

12. Un appareil selon l'une des Revendications 8 à 11, dans lequel des moyens sont fournis afin de faire varier le volume du premier réservoir en réponse à au moins un paramètre du fluide à distribuer.

13. Un appareil selon l'une des Revendications précédentes, dans lequel des moyens (7, 19, 20, 41, 42, 130) sont fournis afin d'alimenter périodiquement le second réservoir (6, 27, 49) en aliquots de volume uniforme du fluide à distribuer.

14. Un appareil selon l'une des Revendications précédentes, dans lequel le second réservoir (6, 27, 49) a une entrée (7, 19, 20, 21, 41, 42, 130) communicant avec une source du fluide à délivrer.

15. Un appareil selon la Revendication 14, dans lequel l'entrée (7, 19, 20, 21, 41, 42, 130) communique avec la source du fluide à distribuer par l'intermédiaire d'une valve unidirectionnelle qui empêche le flux du fluide de s'inverser du réservoir vers la source.

16. Un appareil selon l'une des Revendications précédentes, dans lequel un filtre (9, 22, 43, 51, 102) pour retirer les bactéries est pourvu en aval du second réservoir (6, 27, 49).

17. Un appareil selon l'une des Revendications précédentes, dans lequel un moyen (22, 43, 52, 104) pour retirer l'air du fluide qui est distribué est situé entre le second réservoir (6, 27, 49) et le tube (10, 53, 126).

18. Un appareil selon l'une des Revendications précédentes, dans lequel un manomètre (3) est pourvu en communication avec le premier réservoir (4, 29, 57, 92).

19. Un appareil selon l'une des Revendications précédentes, dans lequel le premier fluide est contenu dans une boucle de courant fermée qui inclue le premier réservoir (4, 29, 57, 92), le premier réservoir communicant dans la boucle avec un dispositif d'alimentation du fluide à fonctionnement périodique (84) pour approvisionner le premier fluide au premier réservoir (4, 29, 37, 92).

20. Un appareil selon l'une des Revendications précédentes, dans lequel des moyens sont fournis pour déterminer le débit du second fluide à travers le tube (10, 53, 126).
